# EUROPEAN PATENT APPLICATION

(11) **EP 0 827 716 A2**
(43) Date of publication of application: **11.03.1998**
(21) Application number: 97115365.5
(22) Date of filing: 04.09.1997
(51) Int. Cl.: A61B 17/36

(54) **Device for cooling skin during laser treatment**

(30) Priority: 04.09.1996 US 707561
(71) Applicant: ESC Medical Systems Ltd., Yokneam 20692 (IL)
(72) Inventor: Karni, Ziv, 46910 Kfar Shemaryahu (IL)
(74) Representative: von Samson-Himmelstjerna, Friedrich R., Dipl.-Phys.

(57) **Abstract**

Summarized, a device and method for cooling the skin of a patient during dermatological laser surgery are disclosed. The device may comprise an annular thermoelectric cooler, used to cool the patient's skin while a pulsed laser beam is directed at the surgical target via the hole of the annulus. Preferred embodiments of the device include a thermally conductive window covering the hole on the cold side of the annulus, a temperature feedback circuit for regulating the electrical current supplied to the device, means for preventing condensation of moisture on the window, and means for synchronizing the operation of the device with the laser pulses. In a variant of the device, the thermoelectric cooler is not annular, but has a thermally conductive window thermally coupled to its cold side. The window itself is used to cool the patient's skin while a pulsed laser beam is directed at the surgical target via the window.

## Description

### FIELD AND BACKGROUND OF THE INVENTION

The present invention relates to laser treatment in dermatological surgery and, more particularly, to a device and method for cooling the skin during that surgery.

Laser beams are commonly used in dermatology, to treat vascular lesions and pigmented lesions. A common side effect of this treatment is thermal injury to the surrounding tissue, as the surrounding tissue is heated to a temperature in excess of about 42°C. The techniques used to minimize this side effect include using a pulsed laser with a pulse duration shorter than the thermal relaxation time of the skin, and using light of a wavelength that is strongly absorbed by the treated tissue. These techniques often are inadequate, because of heat transport to the surrounding tissue, by blood vessels or by thermal conductivity, or because the laser beam spot size is larger than the area to be treated.

Two methods have been used to prevent this thermal injury by directly cooling the skin in the immediate area of the laser beam. Chess, in US Patents 5,057,104, 5,282,797, and 5,486,172, teaches the use of a container of cool transparent stationary or flowing liquid to cool the skin. The laser beam is directed through the container to the lesion to be treated. Nelson et al. ("Epidermal cooling during pulsed laser treatment of selected dermatoses", Proceedings of Medical Applications of Lasers III, 12-16 September 1995, Barcelona, Spain, pp. 32-39) cool the skin by spraying a cryogenic liquid such as tetrafluoroethane on the treated area. Neither method is totally satisfactory. Chess' container is bulky and not suited to laser treatment in confined spaces. The cryogenic liquid of Nelson et al. requires a special insulated reservoir and delivery system, and is not suitable for use near sensitive areas such as the eyes. In addition, it is difficult to monitor the cooling efficacy of the two methods during laser treatment.

There is thus a widely recognized need for, and it would be highly advantageous to have, a device and method, for cooling the skin during laser treatment, which would overcome the disadvantages of presently known systems as described above.

### SUMMARY OF THE INVENTION

According to the present invention there is provided a device for cooling a target being irradiated by a beam of electromagnetic radiation, comprising thermoelectric cooling means having an aperture wherethrough the beam of electromagnetic radiation is directed at the target. Preferred embodiments are defined in the subclaims.

According to the present invention there is provided a method for cooling a target being cut by a beam of electromagnetic radiation, comprising the steps of: (a) providing thermoelectric cooling means having at least one aperture; (b) placing the thermoelectric cooling means in contact with the target; and (c) directing the beam of electromagnetic radiation through the at least one aperture. This method may further comprise the steps of: (a) providing at least one window, transparent to the beam of electromagnetic radiation, having a proximal surface and a distal surface, and being in thermal contact with said thermoelectric cooling means; and (b) placing said distal surface of said at least one window in contact with the target. The at least one window can be made of a material selected from the group consisting of sapphire, quartz and glass. The method may further comprise the step of keeping the at least one window clear. The keeping of the at least one window clear can, for example, be effected by directing dry air at said proximal surface of said at least one window. The method may further comprise the steps of: (a) measuring at least one temperature of the target; and (b) regulating said thermoelectric cooling means based on said at least one temperature measurement. The measuring of the at least one temperature can be effected using at least one thermocouple and/or at least one thermistor. The regulating of said thermoelectric cooling means can be effected using at least one feedback circuit. If the beam of electromagnetic radiation is pulsed, the method may further comprise the step of synchronizing the operation of said thermoelectric cooling means with the pulsed beam.

According to the present invention there is provided a device for cooling a target being irradiated by a beam of electromagnetic radiation, comprising: (a) thermoelectric cooling means; and (b) at least one window, transparent to the electromagnetic radiation, and thermally coupled to the thermoelectric cooling means. Preferred embodiments are defined in the subclaims.

According to the present invention there is provided a method for cooling a target being cut by a beam of electromagnetic radiation, comprising the steps of: (a) providing thermoelectric cooling means; (b) providing at least one window, transparent to the beam of electromagnetic radiation, having a distal surface, and being in thermal contact with the thermoelectric cooling means; (c) placing the distal surface of the at least one window in contact with the target; and (d) directing the beam of electromagnetic radiation through the at least one window. The at least one window may be made of a material selected from the group consisting of sapphire, quartz and glass.

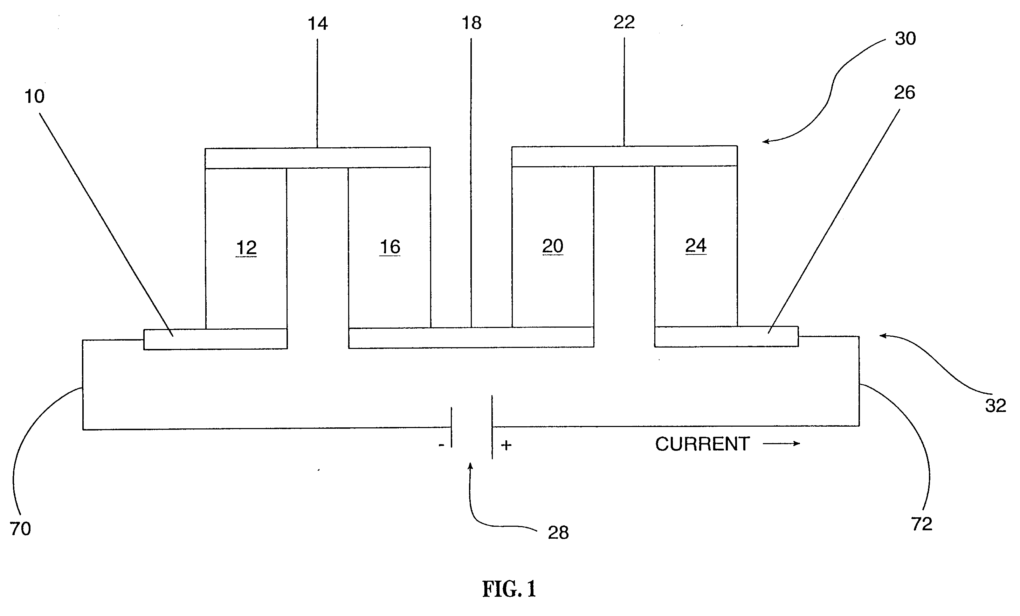

The device of the present invention is based on the Peltier effect, which is the phenomenon of the development of a temperature gradient between the two junctions of an electrical circuit consisting of two dissimilar conductors. Figure 1 shows schematically a commonly used configuration of dissimilar conductors for using this phenomenon to heat or cool a surface. Metallic conductors **10**, **14**, **18**, **22**, and **26**, p-type semiconductors **12** and **20**, and n-type semiconductors **16** and **24** are connected in series as shown. When DC current source **28** introduces electrical current into the circuit in the direction shown, via leads **70** and **72**, one side **30** of the circuit becomes cold, and another side **32** of the circuit becomes hot. Note that if the polarity of DC current source **28** is reversed, then side **32** becomes cold and side **30** becomes hot. Thermoelectric devices, comprising arrays of semiconductors connected as shown in Figure 1, are used in many cooling applications in which space is at a premium, for example, for cooling electronic circuits. Among the manufacturers of such devices is Marlow Industries, Inc., of Dallas TX.

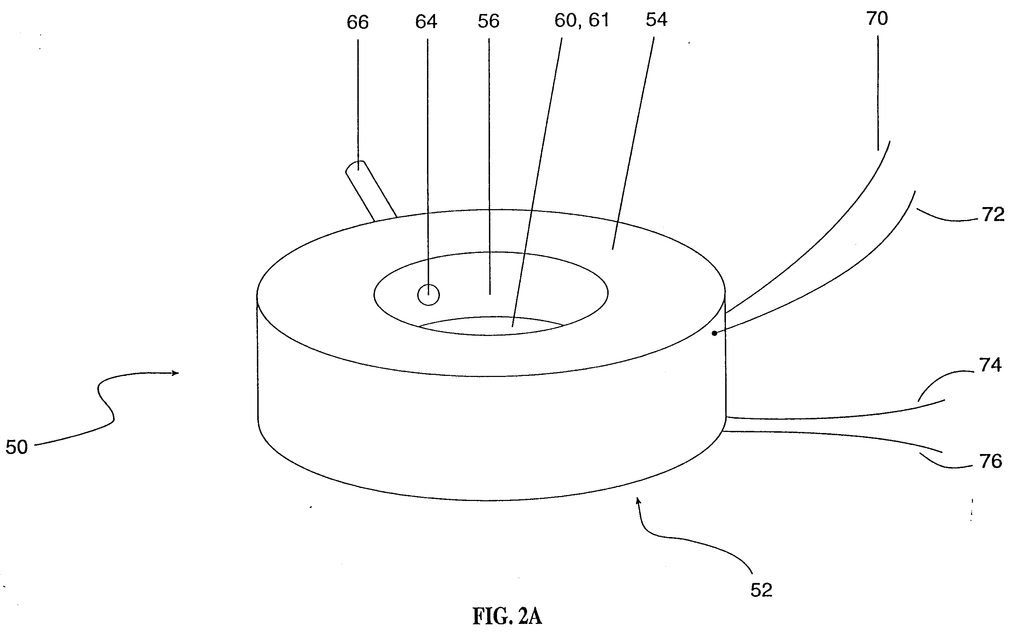

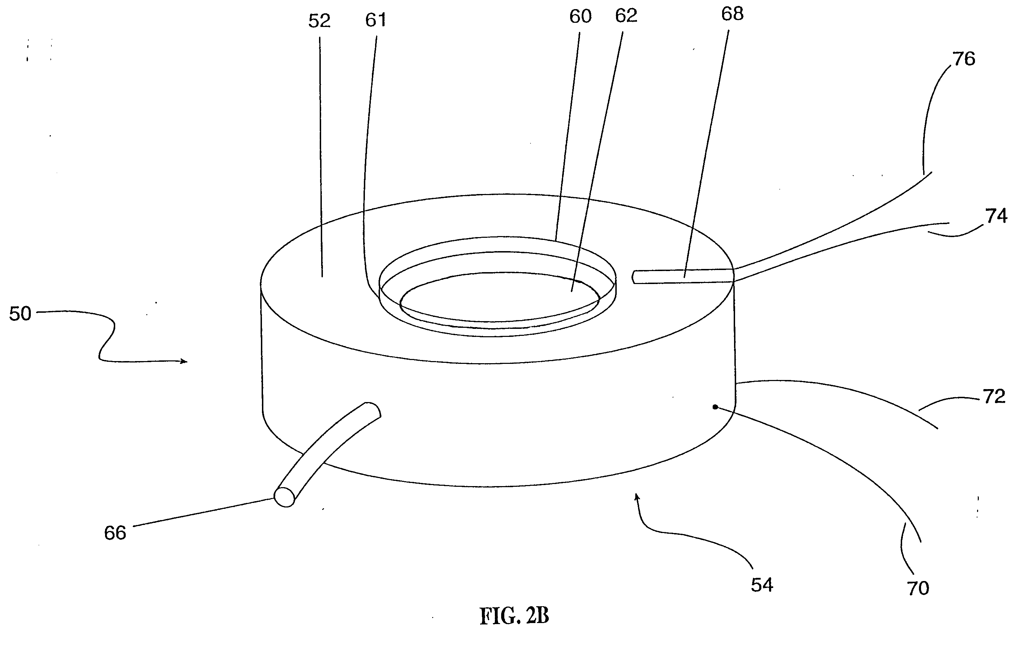

One of the innovations of the present invention is to configure a thermoelectric device in the shape of an annular ring **50**, as shown in Figures 2A and 2B, with the circuitry arranged so that when electrical current is introduced to the device, one flat side **52** of annular ring **50** becomes cold and another flat side **54** of annular ring **50** becomes hot. Cold side **50** is placed on the skin to be irradiated by the laser beam, and the laser beam is directed at the target of irradiation through a central aperture **56** in annular ring **50**. Preferred embodiments of the device of the present invention include a further innovation. Aperture **56** is covered on cold side **50** by a window **60** that is transparent to the laser beam and that is thermally coupled to cold side **50**, thus extending the cooling effect of the device to the portion of the skin covered by window **60**, as well as the portion of the skin covered by cold side **50**.

### BRIEF DESCRIPTION OF THE DRAWINGS

The invention is herein described, by way of example only, with reference to the accompanying drawings, wherein:
FIG. 1 is a schematic diagram of a thermoelectric circuit;
FIG. 2A is a schematic perspective view of a preferred embodiment of the device of the present invention;
FIG. 2B is another schematic perspective view of the preferred embodiment of FIG. 2A;
FIG. 3 is a schematic side view of another preferred embodiment of the device of the present invention;
FIG. 4 is a schematic block diagram of the device of the present invention;
FIG. 5 is a schematic side view of a third preferred embodiment of the device of the present invention.

### DESCRIPTION OF THE PREFERRED EMBODIMENTS

The present invention is of a thermoelectric device which can be used to cool a target while the target is irradiated by a beam of electromagnetic radiation. Specifically, the present invention can be used to cool skin adjacent to the target of dermatological laser surgery.

The principles and operation of a thermoelectric cooling device according to the present invention may be better understood with reference to the drawings and the accompanying description.

Referring now to the drawings, Figures 2A and 2B are schematic perspective views of one preferred embodiment of the device of the present invention. An annular ring **50** encloses thermoelectric circuitry configured so that, when DC current is introduced to that circuitry via leads **70** and **72**, one side **52** of annular ring **50** is cooled and another side **54** of annular ring **50** is heated. Sides **52** and **54** are made of a thermally conducting material. A substantially cylindrical aperture **56** extends through the device from cold side **52** to hot side **54**, so that when cold side **52** is placed against the skin of a patient, therapeutic electromagnetic radiation, for example from a pulsed laser, may be directed through aperture **56** at a target on the patient's skin. A window **60**, having a proximal side **61** and a distal side **62**, covers aperture **56** on cold side **52**. Window **60** is made of a material that is transparent to the electromagnetic radiation. Proximal side **61** of window **60** is attached to cold side **52** of annular ring **50**, and window **60** is coupled thermally to cold side **52**, so that the cooling effect of cold side **52** extends to the skin that is in contact with distal side **62** of window **60**. Preferably, window **60** is made of a material characterized by a high thermal conductivity, that is, a thermal conductivity greater than about .001 cal/sec/cm/°C. For applications in which the electromagnetic radiation is visible, near infrared, or mid infrared radiation, window **60** preferably is made of sapphire, which is transparent to that radiation. In applications restricted to visible or near infrared radiation, quartz or glass may be used for window **60**.

A port **64** in aperture **56** is connected to a tube **66**. Dry air is introduced to aperture **56** via tube **66** and port **64**, to prevent condensation of moisture on proximal side **61** of window **60**, thereby keeping window **60** clear. A temperature sensor **68** is attached to cold side **52** for monitoring the temperature of the skin adjacent to cold side **52**. Temperature sensor **68**, which may be a thermocouple or a thermistor, is connected to measurement circuitry via leads **74** and **76**.

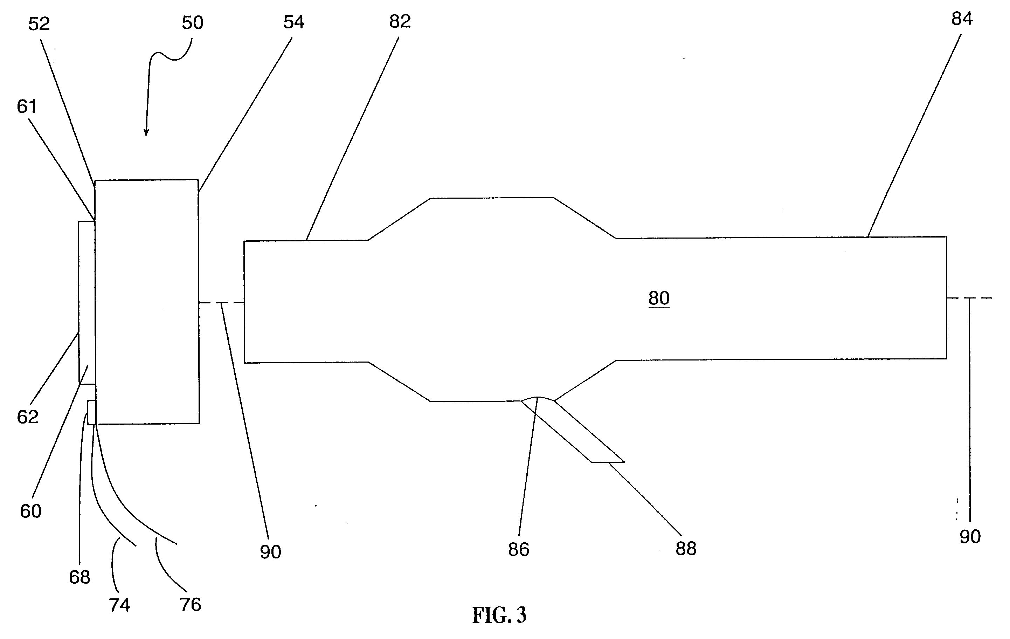

Figure 3 shows a schematic side view of another preferred embodiment of the device of the present invention. In this preferred embodiment, annular ring **50** lacks port **64** and tube **66**. Instead, the device includes a hollow, tubular hand piece **80** having a proximal end **84** and a distal end **82**. Distal end **82** is substantially cylindrical, having an outside diameter substantially equal to the inside diameter of aperture **56**. In use, distal end **82** is inserted into aperture **56**. A beam of electromagnetic radiation travels along an optical path **90** through hand piece **80** and window **60**. The operator holds hand piece **80** so that cold side **52** and window **60** are in contact with the target area of the patient's skin. Hand piece **80** also has a port **86** and a tube **88**, through which dry air is introduced to hand piece **80** to keep moisture from condensing on proximal surface **61** of window **60**.

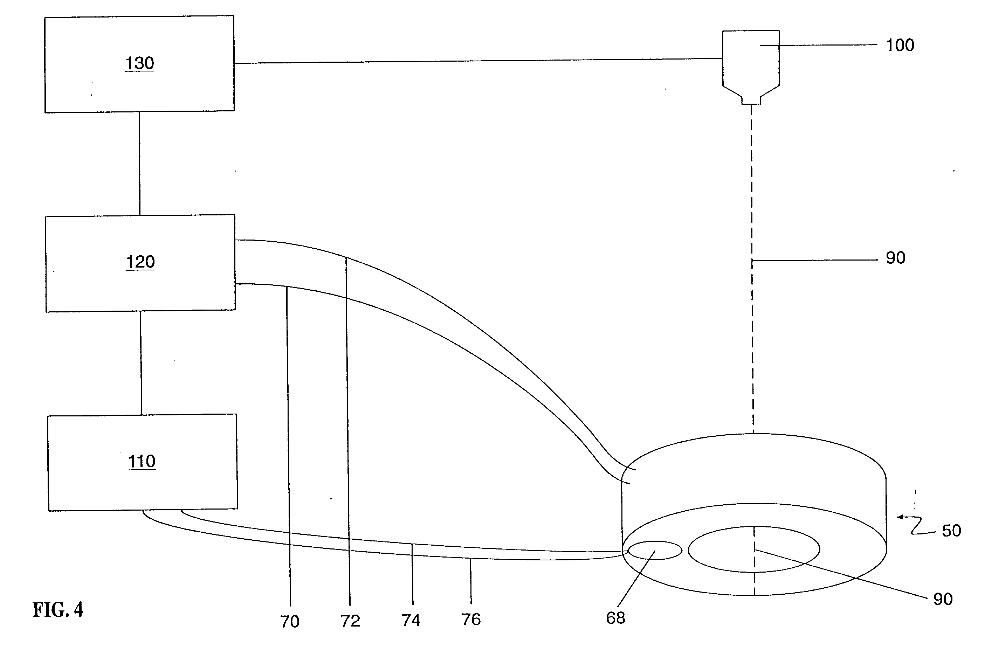

Figure 4 shows a schematic block diagram of these preferred embodiments of the device of the present invention, as they are used in conjunction with a pulsed laser **100** as a source of the electromagnetic radiation. Leads **70** and **72** go to a DC power supply **120**. Both laser **100** and DC power supply **120** are controlled by a controller **130**, which synchronizes power supply **120** with the pulses of laser **100** so that power supply **120** energizes the thermoelectric circuit of the device of the present invention only while cooling is needed, i.e., only while laser **100** actually emits radiation and for a short time afterwards. Leads **74** and **76** go to a feedback circuit **110**, which regulates the electrical current supplied to leads **70** and **72** to keep the temperature of the patient's skin, as measured by temperature sensor **68**, within acceptable bounds. If the temperature rises above about 35°C, feedback circuit **110** increases the current to increase the cooling. If the temperature falls below about 10°C, feedback circuit **110** decreases the current to decrease the cooling.

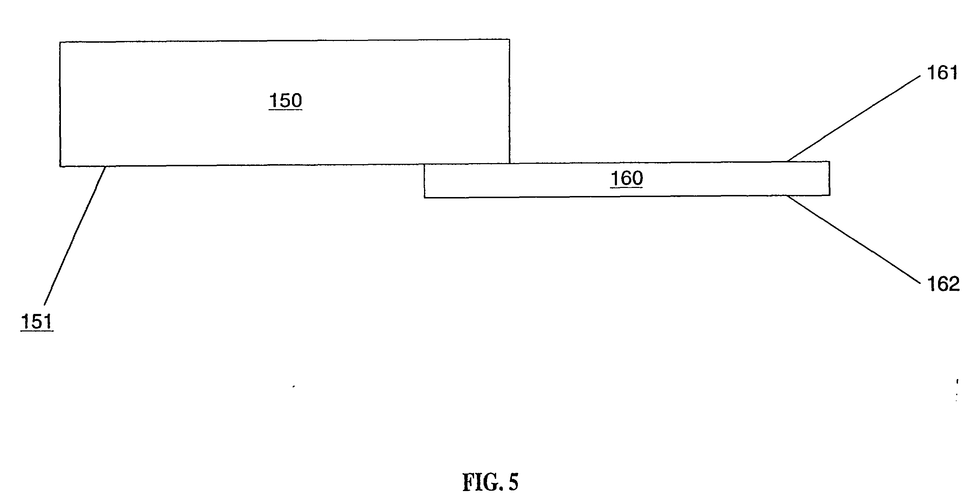

Figure 5 shows a schematic side view of a third preferred embodiment of the device of the present invention. In this embodiment, a window **160** is rigidly attached to a cold side **151** of a thermoelectric cooling device **150** which need not be annular in shape. Window **160** projects to the side of cooling device **150** as shown. Like window **60**, window **160** preferably is made of a transparent material of high (greater than about .01 cal/sec/cm/°C) thermal conductivity, such as sapphire or quartz. In use, distal side **162** of window **160** is placed in contact with the patient's skin, and therapeutic electromagnetic radiation is directed at a target on the patient's skin via proximal side **161** of window **160**. This preferred embodiment is suitable for cooling targets of laser treatment in restricted areas that are too confined to allow the use of the other preferred embodiments.

Summarized, a device and method for cooling the skin of a patient during dermatological laser surgery are disclosed. The device may comprise an annular thermoelectric cooler, used to cool the patient's skin while a pulsed laser beam is directed at the surgical target via the hole of the annulus. Preferred embodiments of the device include a thermally conductive window covering the hole on the cold side of the annulus, a temperature feedback circuit for regulating the electrical current supplied to the device, means for preventing condensation of moisture on the window, and means for synchronizing the operation of the device with the laser pulses. In a variant of the device, the thermoelectric cooler is not annular, but has a thermally conductive window thermally coupled to its cold side. The window itself is used to cool the patient's skin while a pulsed laser beam is directed at the surgical target via the window.

While the invention has been described with respect to a limited number of embodiments, it will be appreciated that many variations, modifications and other applications of the invention may be made.

## Claims

1. A device for cooling a target being irradiated by a beam of electromagnetic radiation, comprising thermoelectric cooling means having an aperture wherethrough the beam of electromagnetic radiation is directed at the target.

2. The device of claim 1, further comprising at least one window, transparent to said electromagnetic radiation, and thermally coupled to said thermoelectric cooling means.

3. The device of claim 2, wherein said at least one window includes at least one material having a high thermal conductivity.

4. The device of claim 3, wherein said at least one material is selected from the group consisting of sapphire, quartz and glass.

5. The device of anyone of claims 2 to 4, further comprising a mechanism for keeping said window clear.

6. The device of claim 5, wherein said mechanism for keeping said window clear includes at least one port for directing dry air at said window.

7. The device of anyone of claims 1 to 6, further comprising:
(a) means for measuring a temperature of the target; and
(b) means, responsive to said means for measuring a temperature of the target, for controlling said thermoelectric cooling means.

8. The device of claim 7, wherein said means for measuring a temperature of the target includes at least one thermocouple.

9. The device of claim 7, wherein said means for measuring a temperature of the target includes at least one thermistor.

10. The device of anyone of claims 7 to 9, wherein said means for controlling said thermoelectric cooling means includes at least one feedback circuit.

11. The device of anyone of claims 1 to 10, wherein the beam of electromagnetic radiation is pulsed, the device further comprising means for synchronizing the cooling of the target with the pulsed beam.

12. A device for cooling a target being irradiated by a beam of electromagnetic radiation, comprising:
(a) thermoelectric cooling means; and
(b) at least one window, transparent to said electromagnetic radiation, and thermally coupled to said thermoelectric cooling means.

13. The device of claim 12, wherein said at least one window includes at least one material having a high thermal conductivity.

14. The device of claim 13, wherein said at least one material is selected from the group consisting of sapphire, quartz and glass.
